# EUROPEAN PATENT APPLICATION

(11) **EP 4 640 696 A1**
(43) Date of publication of application: **29.10.2025**
(21) Application number: 24764163.2
(22) Date of filing: 27.02.2024
(51) Int. Cl.: C07K 14/34, C12N 15/77, C12P 13/14

(54) **VARIANT POLYPEPTIDE AND METHOD FOR PRODUCING L-GLUTAMIC ACID USING SAME**

(30) Priority: 27.02.2023 KR 20230025828
(71) Applicant: CJ CheilJedang Corporation, Seoul 04560 (KR)
(72) Inventor: LEE, Heeseok, Seoul 04560 (KR); KANG, Tae-Gu, Seoul 04560 (KR); CHOI, Sun Hyoung, Seoul 04560 (KR); YOON, Byoung Hoon, Seoul 04560 (KR)
(74) Representative: Meissner Bolte Partnerschaft mbB
(86) International application number: PCT/KR2024/002491
(87) International publication number: WO 2024/181764

(57) **Abstract**

The present disclosure relates to a novel variant polypeptide; a polynucleotide encoding the variant polypeptide; a microorganism, which comprises the variant polypeptide, the polynucleotide encoding the variant polypeptide, or a vector comprising the polynucleotide; a method for producing L-glutamic acid, comprising culturing the microorganism in a medium; and use of the variant polypeptide or microorganism for the production of L-glutamic acid.

## Description

### [Technical Field]

The present disclosure relates to a novel variant polypeptide; a polynucleotide encoding the variant polypeptide; a microorganism, which comprises the variant polypeptide, a polynucleotide encoding the variant polypeptide, or a vector comprising the polynucleotide; a method for producing L-glutamic acid, including culturing the microorganism in a medium; and the use of the variant polypeptide or microorganism for the production of L-glutamic acid.

### [Background Art]

L-glutamic acid, which is a representative amino acid produced by fermentation, has a distinctive taste and thus is one of the important amino acids used broadly not only in the food industry but also in the fields of medicine, other animal feed, *etc.*

Examples of conventional methods for producing L-glutamic acid include a fermentation method using coryneform bacteria including microorganisms of the genus *Brevibacterium, Corynebacterium,* and variants thereof ("Amino Acid Fermentation", Gakkai Shuppan Center, pp. 195-215, 1986); and a method of using *Escherichia coli,* and microorganisms belonging to *Bacillus, Streptomyces, Penicillium, Klebsiella, Erwinia, Pantoea, etc.* (U.S. Application Publication No. 3220929, U.S. Pat. No. 6682912).

Additionally, various studies have been conducted for efficiently producing amino acids; for example, efforts have been made to develop microorganisms or fermentation process technologies for producing amino acids with high efficiency. Particularly, approaching methods for specific to target materials have been developed, such as enhancement of expression of genes encoding enzymes involved in the biosynthesis of the amino acids in the strain of the genus *Corynebacterium* or deletion of genes unnecessary for the biosynthesis of amino acids (Korean Patent Nos. 10-0924065 and 1208480). In addition to these methods, a method for removing genes that are not involved in the production of amino acids and a method for removing genes whose functions for producing amino acids are not specifically known have also been utilized. However, there is still a growing need to study methods for efficiently producing L-glutamic acid with high yield.

### [Disclosure]

### [Technical Problem]

The present inventors have developed a variant of the major facilitator superfamily transporter, a *Corynebacterium glutamicum* strain including the variant, and a method for producing L-glutamic acid using the strain, thereby completing the present disclosure.

### [Technical Solution]

It is one object of the present disclosure to provide a variant polypeptide, in which the amino acid corresponding to position 351 of SEQ ID NO: 1 is substituted with another amino acid.

In one embodiment, the variant polypeptide may be one in which the amino acid corresponding to position 351 of SEQ ID NO: 1 is substituted with asparagine.

In one embodiment, the variant polypeptide may include a sequence having 80% or more sequence identity with the amino acid sequence of SEQ ID NO: 1.

In another embodiment, the variant polypeptide may consist of an amino acid sequence of SEQ ID NO: 3.

In still another embodiment, the variant polypeptide may have the activity of the major facilitator superfamily transporter.

It is another object of the present disclosure to provide a polynucleotide encoding the variant polypeptide.

It is still another object of the present disclosure to provide a microorganism including the variant polypeptide, a polynucleotide encoding the variant polypeptide, or a vector containing the polynucleotide.

As the microorganism according to any one of the above-described embodiments, the microorganism may further include any one or more variant polypeptides selected from the group consisting of (a) and (b); a polynucleotide encoding the variant polypeptide; or a vector containing the polynucleotide:
wherein (a) a variant polypeptide having the activity of a TetR-family transcriptional regulator in which the amino acid corresponding to position 105 of SEQ ID NO: 5 is substituted with another amino acid; and
(b) a variant polypeptide having the activity of isocitrate lyase in which the amino acid sequences corresponding to positions 336 to 346 of SEQ ID NO: 9 are deleted.

As the microorganism according to any one of the above-described embodiments, (a) the variant polypeptide having the activity of a TetR-family transcriptional regulator may be a variant polypeptide having the activity of a TetR-family transcriptional regulator in which the amino acid corresponding to position 105 of SEQ ID NO: 5 is substituted with leucine.

As the microorganism according to any one of the above-described embodiments, (a) the variant polypeptide having the activity of a TetR-family transcriptional regulator may consist of an amino acid sequence of SEQ ID NO: 7; and (b) the variant polypeptide having the activity of isocitrate lyase may consist of an amino acid sequence of SEQ ID NO: 11.

As the microorganism according to any one of the above-described embodiments, the microorganism may have an increased L-glutamic acid-producing ability compared to a microorganism including a polypeptide of SEQ ID NO: 1 or a polynucleotide encoding the same.

As the microorganism according to any one of the above-described embodiments, the microorganism may be a microorganism of the genus *Corynebacterium.*

As the microorganism according to any one of the above-described embodiments, the microorganism of the genus *Corynebacterium* may be *Corynebacterium glutamicum.*

It is yet another object of the present disclosure to provide a method for producing L-glutamic acid, including culturing the microorganism in a medium.

In one embodiment, the method may further include recovering L-glutamic acid from the cultured microorganism, a culture product of the microorganism, a fermented product of the microorganism, or the cultured medium.

It is even another object of the present disclosure to provide a composition for producing L-glutamic acid, including: the variant polypeptide; a polynucleotide encoding the variant polypeptide; a vector containing the polynucleotide; a microorganism, which includes the variant polypeptide, a polynucleotide encoding the variant polypeptide, or a vector containing the polynucleotide; a culture product of the microorganism; or a combination of two or more thereof.

It is further another object of the present disclosure to provide the use of the variant polypeptide; a polynucleotide encoding the variant polypeptide; a vector containing the polynucleotide; a microorganism, which includes the variant polypeptide, a polynucleotide encoding the variant polypeptide, or a vector containing the polynucleotide; a culture product of the microorganism; or a combination of two or more thereof for the production of L-glutamic acid.

### [Advantageous Effects]

When culturing the microorganism including the variant polypeptide of the present disclosure, L-glutamic acid can be produced with high yield compared to a microorganism having an existing non-modified polypeptide.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety and the level of technical field to which the present disclosure belongs and the contents of the present disclosure will be described more clearly.

One aspect of the present disclosure provides a variant polypeptide, in which the amino acid corresponding to position 351 of SEQ ID NO: 1 is substituted with another amino acid.

As used herein, the term "major facilitator superfamily transporter (cgmA)", which is a global transporter, may be used interchangeably with the term "cgmA", and may be a major facilitator superfamily transporter encoded by the *cgmA* gene, but is not particularly limited thereto.

The gene encoding the major facilitator superfamily transporter may be derived from a microorganism of the genus *Corynebacterium,* and may specifically be *cgmA* derived from *Corynebacterium glutamicum,* but is not limited thereto.

Specifically, the major facilitator superfamily transporter may include, for example, an amino acid sequence of SEQ ID NO: 1, or an amino acid sequence having 60% or more homology or identity thereto, but is not limited as long as it has the activity of the major facilitator superfamily transporter. Specifically, the amino acid sequence may include the amino acid sequence of SEQ ID NO: 1 or an amino acid sequence having at least 60%, 70%, 80%, 85%, 90%, 95%, 96%, 97%, 98%, or 99% or more homology or identity to SEQ ID NO: 1. The sequence of SEQ ID NO: 1 can be obtained from a known database, such as NCBI's GenBank or Kyoto Encyclopedia of Genes and Genomes (KEGG). In one example, the amino acid sequence may be derived from a microorganism of the genus *Corynebacterium* or *Corynebacterium glutamicum,* and more specifically may be a polypeptide/protein including the amino acid sequence represented by SEQ ID NO: 1, but is not limited thereto. Additionally, it is apparent that any accessory protein having an amino acid sequence in which part of the sequence is deleted, modified, substituted, or added may fall within the scope of the present disclosure if the amino acid sequence has such a homology or identity and exhibits an efficacy corresponding to that of the protein.

In addition, the major facilitator superfamily transporter having the amino acid sequence of SEQ ID NO: 1 may be encoded by a polynucleotide, which may have or include a nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, or may consist or essentially consist of a nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, but is not limited thereto.

As used herein, the term "variant" refers to a polypeptide having one or more amino acids different from the amino acid sequence prior to mutation of the variant by conservative substitutions and/or modifications such that the functions and properties of the polypeptide are retained. Such variants may generally be identified by modifying one or more of the amino acid sequences of the polypeptide and evaluating the properties of the modified polypeptide. That is, the ability of the variants may be enhanced, unchanged, or reduced relative to a polypeptide prior to mutation. Further, some variants may include those in which one or more regions, such as an N-terminal leader sequence or transmembrane domain, have been removed. Further, other variants may include those in which a region has been removed from the N- and/or C-terminal of a mature protein. The term "variant" may be used interchangeably with terms such as modification, modified protein, mutant, mutein, divergent, variant, *etc.* without limitation, as long as the terms are used to indicate variation.

Additionally, the variant may also include deletion or addition of amino acids that have minimal influence on the properties and secondary structure of a polypeptide. For example, the polypeptide may be conjugated with a signal (or leader) sequence at the N-terminal involved in the transfer of proteins co-translationally or posttranslationally. Further, the polypeptide may also be conjugated with another sequence or linker to identify, purify, or synthesize the polypeptide.

The variant polypeptide of the present disclosure may be a variant polypeptide in which the amino acid corresponding to position 351 of SEQ ID NO: 1 is substituted with another amino acid, but is not limited thereto.

In one embodiment, the variant polypeptide of the present disclosure may have a sequence homology of 60% or more and less than 100%, specifically, a sequence homology of 80% or more and less than 100% with the amino acid sequence of SEQ ID NO: 1, but is not limited thereto.

Specifically, the variant polypeptide of the present disclosure may include those in which the amino acid at position 351 from the N-terminus of SEQ ID NO: 1 is substituted with another amino acid, in the amino acid sequence having at least 60%, 70%, 75%, 80%, 85%, 90%, 95%, 96%, 97%, 98%, 99%, 99.5%, 99.7% or 99.9% or more homology or identity with the amino acid sequence represented by SEQ ID NO: 1. Additionally, it is apparent that any variant having an amino acid sequence, in which part of the sequence is deleted, modified, substituted, conservatively substituted, or added, may also fall within the scope of the present disclosure as long as it has such homology or identity and exhibits an efficacy corresponding to that of the variant of the present disclosure.

The "another amino acid" is not limited as long as it is substituted with an amino acid other than the amino acid prior to substitution. Meanwhile, in the present disclosure, when it is expressed that 'a specific amino acid has been substituted', it is apparent that the amino acid is substituted with an amino acid different from the amino acid prior to substitution, even if it is not specifically stated that the amino acid has been substituted with a different amino acid.

The amino acids may generally be classified based on similarity of polarity, charge, solubility, hydrophobicity, hydrophilicity, and/or amphipathic nature of a residue.

The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chains (electrically charged amino acids) include arginine, lysine, histidine, glutamic acid, and aspartate; and amino acids having uncharged side chains (referred to as uncharged amino acids; neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids can be classified according to their size into 5 groups, starting from the amino acid groups with a relatively small volume, i.e., glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto.

For example, when it is described as "the amino acid corresponding to position 351 of SEQ ID NO: 1 is substituted with another amino acid", it may mean that the amino acid is substituted with asparagine, valine, glycine, alanine, glutamate, phenylalanine, arginine, aspartate, cysteine, glutamine, histidine, proline, serine, tyrosine, lysine, tryptophan, methionine, threonine, or leucine, except isoleucine, but is not limited thereto.

In the present disclosure, although it is described as "a protein having an amino acid sequence described by a specific sequence number", it is apparent that any protein having an amino acid sequence in which part of the sequence is deleted, modified, substituted, conservatively substituted or added can be used in the present disclosure if it has the same or corresponding activity as the protein consisting of the amino acid sequence of the corresponding sequence number. For example, sequence additions upstream or downstream of the amino acid sequences that do not alter the function of the protein, naturally occurring mutations, silent mutation thereof, or conservative substitutions are not excluded, as long as the protein has activity identical or corresponding to the activity of the variant protein, and it will be apparent to those skilled in the art that such sequence additions or mutations belong to the scope of the present disclosure.

The "N^{th} position" of the present disclosure may include the N^{th} position and an amino acid position corresponding to the N^{th} position. Specifically, it may include an amino acid position corresponding to any amino acid residue in a mature polypeptide disclosed in a specific amino acid sequence. The specific amino acid sequence may be the amino acid sequence of SEQ ID NO: 1.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48: 443-453) and Needleman program of the EMBOSS package (EMBOSS: The European Molecular Biology Open Software Suite, Rice et al., 2000, Trends Genet. 16: 276-277), *etc.,* but is not limited thereto, and sequence alignment programs, such as pairwise sequence comparison algorithms, *etc.,* known in the art may be appropriately used.

In one embodiment, the variant polypeptide may be one in which the amino acid corresponding to position 351 of SEQ ID NO: 1 is substituted with one selected from the group consisting of asparagine, valine, glycine, leucine, arginine, alanine, methionine, threonine, glutamine, proline, serine, tryptophan, phenylalanine, histidine, cysteine, tyrosine, lysine, aspartate, and glutamate, but is not limited thereto.

In any one of the above-described embodiments, the variant polypeptide provided herein may be one in which the amino acid corresponding to position 351 of SEQ ID NO: 1 is substituted with an amino acid having a polar or hydrophilic side chain (polar amino acid), i.e., an amino acid selected from serine, threonine, cysteine, tyrosine, asparagine, and glutamine.

In any one of the above-described embodiments, the variant polypeptide provided herein may be one in which the amino acid corresponding to position 351 of SEQ ID NO: 1 is substituted with an amino acid having an uncharged side chain (referred to as uncharged amino acid; neutral amino), i.e., an amino acid selected from glycine, alanine, valine, leucine, methionine, phenylalanine, tryptophan, proline, serine, threonine, cysteine, tyrosine, asparagine, and glutamine.

In any one of the above-described embodiments, the variant polypeptide provided herein may be one in which the amino acid corresponding to position 351 of SEQ ID NO: 1 is substituted with an amino acid selected from cysteine, proline, threonine, aspartic acid, and asparagine.

In any one of the above-described embodiments, the variant polypeptide of the present disclosure may be a polypeptide in which the amino acid corresponding to position 351 of SEQ ID NO: 1 is substituted with asparagine, but is not limited thereto.

For example, the variant polypeptide of the present disclosure may include an amino acid sequence having at least 60%, 70%, 75%, 80%, 85%, 90%, 95%, 96%, 97%, 98%, 99%, 99.5%, 99.7%, or 99.9% or more homology or identity with SEQ ID NO: 1, while asparagine, which is the amino acid corresponding to position 351 in the amino acid sequence represented by SEQ ID NO: 1, is fixed. Additionally, it is apparent that any variant polypeptide having an amino acid sequence, in which part of the sequence is deleted, modified, substituted, or added, may also fall within the scope of the present disclosure as long as the amino acid sequence has such a homology or identity and exhibits an efficacy corresponding to that of the variant polypeptide of the present disclosure.

Meanwhile, those skilled in the art can determine the amino acid corresponding to position 351 of the amino acid sequence of SEQ ID NO: 1 of the present disclosure in any amino acid sequence through sequence alignment known in the art, and even if not separately described herein, it is apparent that the phrase "an amino acid at a particular position in a particular sequence number" obviously includes even "an amino acid at a position corresponding" thereto in any polynucleotide sequence.

In another embodiment, the variant polypeptide may consist of an amino acid sequence of SEQ ID NO: 3.

Specifically, the variant polypeptide of the present disclosure may have or include the amino acid sequence of SEQ ID NO: 3 or an amino acid sequence having at least 60%, 70%, 80%, 85%, 90%, 95%, 96%, 97%, 98%, or 99% or more homology or identity thereto, may consist of the amino acid sequence above, or may essentially consist of the amino acid sequence above.

For example, it may be a case of sequence additions or deletions that do not alter the function of the variant polypeptide of the present disclosure, naturally occurring mutations, silent mutations thereof, or conservative substitutions at the N-terminus, C-terminus and/or within the amino acid sequences.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids can be classified into amino acids with electrically charged side chains and amino acids with uncharged side chains, and the amino acids with electrically charged side chains include aspartic acid, glutamic acid, lysine, arginine, and histidine, and the amino acids with uncharged side chains can be further classified into nonpolar amino acids or polar amino acids. The non-polar amino acids include glycine, alanine, valine, leucine, isoleucine, methionine, phenylalanine, tryptophan, proline, and the polar amino acids include serine, threonine, cysteine, tyrosine, asparagine, and glutamine. Typically, conservative substitutions have little or no effect on the activity of the resulting polypeptide. Typically, conservative substitutions may have little or no effect on the activity of the protein or polypeptide. In one embodiment, the variant polypeptide may have the activity of the major facilitator superfamily transporter.

Another aspect of the present disclosure provides a polynucleotide encoding the variant polypeptide.

As used herein, the term "polynucleotide", which is a polymer of nucleotides composed of nucleotide monomers connected in a lengthy chain by a covalent bond, is a DNA or RNA strand having at least a certain length. More specifically, it may mean a polynucleotide fragment encoding the variant.

The polynucleotide encoding the variant polypeptide of the present disclosure may include any polynucleotide sequence encoding the variant polypeptide of the present disclosure without limitation. For example, the polynucleotide encoding the variant polypeptide of the present disclosure may be a polynucleotide sequence encoding the amino acid sequence of the variant polypeptide of the present disclosure, but is not limited thereto.

For example, it may include a nucleic acid sequence encoding the amino acid sequence represented by SEQ ID NO: 3. In one example, the polynucleotide of the present disclosure may have or include SEQ ID NO: 4. In addition, the polynucleotide of the present disclosure may consist or essentially consist of SEQ ID NO: 4.

The polynucleotide of the present disclosure may undergo various modifications in the coding region within the scope that does not change the amino acid sequence of the variant of the present disclosure, due to codon degeneracy or in consideration of the codons preferred in an organism in which the variant of the present disclosure is to be expressed. Therefore, it will be apparent that a polynucleotide that may be translated into the polypeptide consisting of the amino acid sequence of the variant of the present disclosure or a polypeptide having a homology or identity thereto by codon degeneracy may also be included. For example, the polynucleotide of the present disclosure may be SEQ ID NO: 4 or a degenerated sequence thereof.

For example, the polynucleotide of the present disclosure may include those in which the codon encoding isoleucine, the amino acid corresponding to position 1052 of SEQ ID NO: 2, is substituted with a codon encoding another amino acid other than isoleucine, e.g., asparagine, in the nucleotide sequence having 60%, 70%, 75%, 80%, 85%, 90%, 95%, 96%, 97%, 98%, 99%, 99.5%, 99.7%, or 99.9% or more homology or identity with the sequence of SEQ ID NO: 2, but is not limited thereto. Additionally, it is apparent that any variant having a polynucleotide sequence, in which part of the sequence is deleted, modified, substituted, conservatively substituted, or added, may also fall within the scope of the present disclosure as long as the polynucleotide sequence has such a homology or identity and encodes the amino acid sequence of the variant polypeptide of the present disclosure.

In another example, the polynucleotide of the present disclosure may have or include a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 4, or may consist or essentially consist of a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 4, but is not limited thereto. Additionally, the sequence having such a homology or identity may be one in which the codon corresponding to position 351 of SEQ ID NO: 3, encoded by SEQ ID NO: 4, is fixed with a codon encoding asparagine.

In addition, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions without limitation.

The "stringent conditions" refer to conditions which allow the specific hybridization between polynucleotides. Such conditions are disclosed in detail in the literature (*e.g*., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, i.e., polynucleotides having 60% or more, 70% or more, 75% or more, 80% or more, 85% or more, 90% or more, 95% or more, 96% or more, 97% or more, 98% or more, or 99% or more hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing polynucleotides depends on the length and degree of complementarity of the polynucleotides, and these variables are well known in the art (see Sambrook et al., supra).

As used herein, the term "homology" or "identity" refers to a degree of similarity between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et at, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" used herein indicates a measure of relevance between sequences.

Still another aspect of the present disclosure provides a vector containing the polynucleotide of the present disclosure. The vector may be an expression vector for expressing the polynucleotide in a microorganism, but is not limited thereto.

As used herein, the term "vector" may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable microorganism, the vector may replicate or function independently from the host genome, or may integrate into the genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target protein may be inserted into the chromosome through a vector for intracellular chromosomal insertion. The insertion of the polynucleotide into the chromosome may be performed by any method known in the art, for example, by homologous recombination, but is not limited thereto. **The** vector may further include a selection marker to confirm the insertion into the chromosome. **The** selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a microorganism so that the polypeptide encoded by the polynucleotide can be expressed in the microorganism. As long as the transformed polynucleotide can be expressed in the microorganism, it does not matter whether the transformed polynucleotide is integrated into the chromosome of the microorganism and located therein or located extrachromosomally, and both cases can be included. Further, the polynucleotide may include DNA and/or RNA encoding the target polypeptide. The polynucleotide may be introduced in any form, as long as it can be introduced into the microorganism and expressed therein. For example, the polynucleotide may be introduced into the microorganism in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a microorganism as it is and operably linked to sequences required for expression in the microorganism, but is not limited thereto.

Further, as used herein, the term "operably linked" means that the polynucleotide sequence is functionally linked to a promoter sequence that initiates and mediates transcription of the polynucleotide encoding the target variant of the present disclosure.

Yet another aspect of the present disclosure provides a microorganism including the variant polypeptide, a polynucleotide encoding the variant polypeptide, or a vector containing the polynucleotide.

In one embodiment, the microorganism may be a microorganism having an L-glutamic acid-producing ability.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product. In the present disclosure, the "microorganism" and "strain" have the same meaning and may be used interchangeably without limitation.

As used herein, the term "L-glutamic acid" or "L-glutamate" refers to a kind of amino acid and is classified as a non-essential amino acid. L-glutamic acid is known to be the most common excitatory neurotransmitter in the central nervous system and since L-glutamic acid has an umami taste, it is widely used as a flavor enhancer since the monosodium glutamate (MSG) has been developed therefrom.

As used herein, the term "microorganism for producing L-glutamic acid", which is a prokaryotic or eukaryotic microbial strain capable of producing L-glutamic acid in an organism, may include both a microorganism, in which an L-glutamic acid-producing ability has been imparted to a parent strain having no L-glutamic acid-producing ability, or a microorganism that endogenously has an L-glutamic acid-producing ability. The L-glutamic acid-producing ability may be imparted or enhanced by the improvement of species.

In one embodiment, the microorganism of the present disclosure may be a microorganism naturally having the variant polypeptide of the present disclosure or an L-glutamic acid-producing ability; or a microorganism in which the variant of the present disclosure or a polynucleotide encoding the same (or a vector containing the polynucleotide) has been introduced into a parent strain not having the variant polypeptide of the present disclosure or an L-glutamic acid-producing ability and/or in which an L-glutamic acid-producing ability has been imparted, but is not limited thereto.

In one embodiment, the microorganism of the present disclosure may include both a microorganism, which includes the variant polypeptide sequence of the present disclosure due to the mutation of a gene encoding the variant polypeptide of the present disclosure on the chromosome, and/or a microorganism, which includes the variant polypeptide of the present disclosure by introducing a vector containing a polynucleotide encoding the variant polypeptide of the present disclosure, but is not limited thereto.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. For example, the non-modified microorganism may refer to a strain into which the variant polypeptide disclosed herein is not introduced, or a strain before the introduction thereof. The "non-modified microorganism" may be used interchangeably with "strain before modification", "microorganism before modification", "non-mutant strain", "non-modified strain", "non-mutant microorganism", or "reference microorganism".

The microorganism having an L-glutamic acid-producing ability of the present disclosure may be a microorganism including any one or more of the variant of the present disclosure, the polynucleotide of the present disclosure, and the vector containing the polynucleotide of the present disclosure; a microorganism modified to express the variant of the present disclosure or the polynucleotide of the present disclosure; a microorganism (*e.g*., a recombinant strain) expressing the variant of the present disclosure or the polynucleotide of the present disclosure; or a microorganism (*e.g*., a recombinant strain) having the variant activity of the present disclosure, but is not limited thereto.

In one example, the strain of the present disclosure, which is a cell or microorganism transformed with the polynucleotide of the present disclosure or a vector containing the polynucleotide encoding the variant of the present disclosure to express the variant of the present disclosure, may include all microorganisms capable of producing L-glutamic acid, including the variant of the present disclosure. For example, the microorganism of the present disclosure may be a recombinant strain whose L-glutamic acid-producing ability is increased by introducing the polynucleotide encoding the variant of the present disclosure into a natural wild-type microorganism or a microorganism having an L-glutamic acid-producing ability, thereby expressing the variant polypeptide. The recombinant strain with an increased L-glutamic acid-producing ability may be a microorganism having an increased L-glutamic acid-producing ability compared to a natural wild-type microorganism or a non-modified microorganism of major facilitator superfamily transporter (*e.g*., a microorganism expressing a wild-type major facilitator superfamily transporter or a microorganism not expressing the variant of the present disclosure), but is not limited thereto. In one example, the microorganism with an increased L-glutamic acid-producing ability may be a microorganism having an increased L-glutamic acid-producing ability compared to a microorganism including the polypeptide of SEQ ID NO: 1 or a polynucleotide encoding the same, but is not limited thereto. In one example, the non-modified microorganism, which is the target strain for comparing the increase in the L-glutamic acid-producing ability, may be ATCC13869 or ATCC13032, which is a wild-type *Corynebacterium glutamicum* strain; or KFCC11074 strain (KR 10-0292299 B1), which is a glutamic-producing strain, but is not limited thereto.

The microorganism may include all microorganisms capable of expressing the variant polypeptide of the present disclosure by various known methods in addition to the introduction of the nucleic acid or vector.

In one example, the microorganism having an increased L-glutamic acid-producing ability may have an increased L-glutamic acid-producing ability by about 1% or more, specifically about 1% or more, about 2% or more, about 3% or more, about 5% or more, about 10% or more, about 15% or more, about 20% or more, about 25% or more, about 30% or more, about 35% or more, about 40% or more, about 45% or more, about 46% or more, about 47% or more, about 48% or more, about 49% or more, about 50% or more, about 51% or more, or about 52% or more (the upper limit is not particularly limited, for example, about 200% or less, about 150% or less, about 100% or less, about 90% or less, about 80% or less, about 70% or less, about 65% or less, about 60% or less, about 55% or less, or about 53% or less) as compared to the L-glutamic acid-producing ability of a parent strain before modification or a non-modified microorganism, but is not limited thereto, as long as it has an increased positive (+) value compared to the production ability of a parent strain before modification or a non-modified microorganism. In another example, the recombinant strain having an increased L-glutamic acid-producing ability may have an increased L-glutamic acid-producing ability by about 1.01 times or more, about 1.02 times or more, about 1.03 times or more, about 1.05 times or more, about 1.1 times or more, about 1.15 times or more, about 1.20 times or more, about 1.25 times or more, about 1.30 times or more, about 1.35 times or more, about 1.40 times or more, about 1.45 times or more, about 1.46 times or more, about 1.47 times or more, about 1.48 times or more, about 1.49 times or more, about 1.50 times or more, about 1.51 times or more, or about 1.52 times or more (the upper limit is not particularly limited, for example, about 10 times or less, about 5 times or less, about 3 times or less, about 2 times or less, or about 1.53 times or less) as compared to that of a parent strain before modification or a non-modified microorganism, but is not limited thereto. As used herein, the term "about" refers to a range which includes all of ±0.5, ±0.4, ±0.3, ±0.2, ±0.1, *etc.,* and includes all of the values equivalent to those which come immediately after the term "about" or those in a similar range, but the range is not limited thereto.

As the microorganism according to any one of the above-described embodiments, the microorganism of the present disclosure may be a microorganism belonging to *Corynebacteria sp. Escherichia sp., Erwinia sp., Serratia sp., Providencia sp., Pseudomonas sp., Leptospira sp., Salmonella sp., Brevibacteria sp., Hypomononas sp., Chromobacterium sp.,* and *Norcardia sp.,* or fungi or yeasts, specifically, a microorganism belonging to *Corynebacteria sp.,* but is not limited thereto.

In one example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism belonging to *Corynebacteria sp.,* more specifically, *Corynebacterium glutamicum,* but is not limited thereto.

The microorganism having an L-glutamic acid-producing ability of the present disclosure may further include any one or more variant polypeptides selected from the group consisting of (a) and (b) below; a polynucleotide encoding the variant polypeptide; or a vector containing the polynucleotide, but is not limited thereto:
wherein (a) a variant polypeptide having the activity of a TetR-family transcriptional regulator in which the amino acid corresponding to position 105 of SEQ ID NO: 5 is substituted with another amino acid; and
(b) a variant polypeptide having the activity of isocitrate lyase in which the amino acid sequences corresponding to positions 336 to 346 of SEQ ID NO: 9 are deleted.

In one example, the microorganism having an L-glutamic acid-producing ability of the present disclosure may further include a variant polypeptide having the activity of a TetR-family transcriptional regulator in which the amino acid corresponding to position 105 of SEQ ID NO: 5 is substituted with another amino acid; a polynucleotide encoding the variant polypeptide; or a vector containing the polynucleotide, but is not limited thereto.

As used herein, the term "variant polypeptide having the activity of a TetR-family transcriptional regulator" may refer to a variant polypeptide having the activity of a TetR-family transcriptional regulator, which includes one or more amino acid substitutions in the amino acid sequence of a polypeptide having the activity of a TetR-family transcriptional regulator; or a variant of a polypeptide having the activity of a TetR-family transcriptional regulator, which includes one or more amino acid substitutions in the parent sequence, the amino acid sequence of a polypeptide having the activity of a TetR-family transcriptional regulator.

As used herein, the term "TetR-family transcriptional regulator (mmpLR)", which is a global regulator having activities involved in excretion, cell division, and stress response, may be used interchangeably with the term "mmpLR", and may be a TetR-family transcriptional regulator encoded by the *mmpLR* gene, but is not particularly limited thereto.

The gene encoding the TetR-family transcriptional regulator may be derived from a microorganism of the genus *Corynebacterium,* and may specifically be *mmpLR* derived from *Corynebacterium glutamicum,* but is not limited thereto.

Specifically, the TetR-family transcriptional regulator may include, for example, an amino acid sequence of SEQ ID NO: 5, or an amino acid sequence having 60% or more homology or identity thereto, but is not limited as long as it has the activity of the TetR-family transcriptional regulator. Specifically, the amino acid sequence may include the amino acid sequence of SEQ ID NO: 5 or an amino acid sequence having at least 60%, 70%, 80%, 85%, 90%, 95%, 96%, 97%, 98%, or 99% or more homology or identity to SEQ ID NO: 5. The sequence of SEQ ID NO: 5 can be obtained from a known database, such as NCBI's GenBank or Kyoto Encyclopedia of Genes and Genomes (KEGG). In one example, the amino acid sequence may be derived from a microorganism of the genus *Corynebacterium* or *Corynebacterium glutamicum,* and more specifically may be a polypeptide/protein including the amino acid sequence represented by SEQ ID NO: 5, but is not limited thereto. Additionally, it is apparent that any accessory protein having an amino acid sequence in which part of the sequence is deleted, modified, substituted, or added may fall within the scope of the present disclosure if the amino acid sequence has such a homology or identity and exhibits an efficacy corresponding to that of the protein.

In addition, the TetR-family transcriptional regulator having the amino acid sequence of SEQ ID NO: 5 may be encoded by a polynucleotide, which may have or include a nucleotide sequence of SEQ ID NO: 6, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 6, or may consist or essentially consist of a nucleotide sequence of SEQ ID NO: 6, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 6, but is not limited thereto.

The variant polypeptide having the activity of the TetR-family transcriptional regulator of the present disclosure may be a variant polypeptide having the activity of a TetR-family transcriptional regulator in which the amino acid corresponding to position 105 of SEQ ID NO: 5 is substituted with another amino acid, but is not limited thereto.

In one embodiment, the variant polypeptide having the activity of the TetR-family transcriptional regulator of the present disclosure may have a sequence homology of 60% or more and less than 100%, specifically, a sequence homology of 80% or more and less than 100% with the amino acid sequence of SEQ ID NO: 5, but is not limited thereto.

Specifically, the variant polypeptide having the activity of the TetR-family transcriptional regulator of the present disclosure may include those in which the amino acid at position 105 from the N-terminus of SEQ ID NO: 5 is substituted with another amino acid, in the amino acid sequence having at least 60%, 70%, 75%, 80%, 85%, 90%, 95%, 96%, 97%, 98%, 99%, 99.5%, 99.7% or 99.9% or more homology or identity with the amino acid sequence represented by SEQ ID NO: 5. Additionally, it is apparent that any variant having an amino acid sequence, in which part of the sequence is deleted, modified, substituted, conservatively substituted, or added, may also fall within the scope of the present disclosure as long as it has such a homology or identity and exhibits an efficacy corresponding to that of the variant of the present disclosure.

In one embodiment, the variant polypeptide having the activity of the TetR-family transcriptional regulator of the present disclosure may be a polypeptide in which the amino acid corresponding to position 105 of SEQ ID NO: 5 is substituted with leucine, but is not limited thereto.

For example, the variant polypeptide having the activity of the TetR-family transcriptional regulator of the present disclosure may include an amino acid sequence having at least 60%, 70%, 75%, 80%, 85%, 90%, 95%, 96%, 97%, 98%, 99%, 99.5%, 99.7%, or 99.9% or more homology or identity with SEQ ID NO: 5, while leucine, which is the amino acid corresponding to position 105 in the amino acid sequence represented by SEQ ID NO: 5, is fixed. Additionally, it is apparent that any variant polypeptide having an amino acid sequence, in which part of the sequence is deleted, modified, substituted, or added, may also fall within the scope of the present disclosure as long as the amino acid sequence has such a homology or identity and exhibits an efficacy corresponding to that of the variant polypeptide of the present disclosure.

In another embodiment, the variant polypeptide having the activity of the TetR-family transcriptional regulator may consist of an amino acid sequence of SEQ ID NO: 7.

Specifically, the variant polypeptide having the activity of the TetR-family transcriptional regulator of the present disclosure may have or include the amino acid sequence of SEQ ID NO: 7 or an amino acid sequence having at least 60%, 70%, 80%, 85%, 90%, 95%, 96%, 97%, 98%, or 99% or more homology or identity to SEQ ID NO: 7, may consist of the amino acid sequence above, or may essentially consist of the amino acid sequence above.

For example, it may be a case of sequence additions or deletions that do not alter the function of the variant polypeptide of the present disclosure, naturally occurring mutations, silent mutations thereof, or conservative substitutions at the N-terminus, C-terminus and/or within the amino acid sequences.

The polynucleotide encoding the variant polypeptide having the activity of the TetR-family transcriptional regulator of the present disclosure may include those in which the codon encoding proline, the amino acid corresponding to position 314 of SEQ ID NO: 6, is substituted with a codon encoding another amino acid other than proline, *e.g*., leucine, in the nucleotide sequence having 60%, 70%, 75%, 80%, 85%, 90%, 95%, 96%, 97%, 98%, 99%, 99.5%, 99.7%, or 99.9% or more homology or identity with the sequence of SEQ ID NO: 6, but is not limited thereto. Additionally, it is apparent that any variant having a polynucleotide sequence, in which part of the sequence is deleted, modified, substituted, conservatively substituted, or added, may also fall within the scope of the present disclosure as long as the polynucleotide sequence has such a homology or identity and encodes the amino acid sequence of the variant polypeptide having the activity of the TetR-family transcriptional regulator of the present disclosure.

In another example, the polynucleotide encoding the variant polypeptide having the activity of the TetR-family transcriptional regulator of the present disclosure may have or include a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 8, or may consist or essentially consist of a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 8, but is not limited thereto. Additionally, the sequence having such a homology or identity may be one in which the codon corresponding to position 105 of SEQ ID NO: 7, encoded by SEQ ID NO: 8, is fixed with a codon encoding leucine.

In one example, the microorganism having an L-glutamic acid-producing ability of the present disclosure may further include a variant polypeptide having the activity of isocitrate lyase in which the amino acid sequences corresponding to positions 336 to 346 of SEQ ID NO: 9 are deleted; a polynucleotide encoding the variant polypeptide; or a vector containing the polynucleotide, but is not limited thereto.

As used herein, the term "a variant polypeptide having the activity of isocitrate lyase" may refer to a variant polypeptide having the activity of isocitrate lyase, which includes one or more amino acid substitutions in the amino acid sequence of a polypeptide having the activity of isocitrate lyase; or a variant of a polypeptide having the activity of isocitrate lyase, which includes one or more amino acid substitutions in the parent sequence, the amino acid sequence of a polypeptide having the activity of isocitrate lyase.

As used herein, the term "isocitrate lyase (aceA)", which is an enzyme that produces succinate using isocitrate as a substrate, may be used interchangeably with the term "aceA", and may be an isocitrate lyase encoded by the *aceA* gene, but is not particularly limited thereto.

The gene encoding the isocitrate lyase may be derived from a microorganism of the genus *Corynebacterium,* and may specifically be aceA derived from *Corynebacterium glutamicum,* but is not limited thereto.

Specifically, the isocitrate lyase may include, for example, an amino acid sequence of SEQ ID NO: 9, or an amino acid sequence having 60% or more homology or identity thereto, but is not limited as long as it has the activity of the isocitrate lyase. Specifically, the amino acid sequence may include the amino acid sequence of SEQ ID NO: 9 or an amino acid sequence having at least 60%, 70%, 80%, 85%, 90%, 95%, 96%, 97%, 98%, or 99% or more homology or identity to SEQ ID NO: 9. The sequence of SEQ ID NO: 9 can be obtained from a known database, such as NCBI's GenBank or Kyoto Encyclopedia of Genes and Genomes (KEGG). In one example, the amino acid sequence may be derived from a microorganism of the genus *Corynebacterium* or *Corynebacterium glutamicum,* and more specifically may be a polypeptide/protein including the amino acid sequence represented by SEQ ID NO: 9, but is not limited thereto. Additionally, it is apparent that any accessory protein having an amino acid sequence in which part of the sequence is deleted, modified, substituted, or added may fall within the scope of the present disclosure if the amino acid sequence has such a homology or identity and exhibits an efficacy corresponding to that of the protein.

In addition, the isocitrate lyase having the amino acid sequence of SEQ ID NO: 9 may be encoded by a polynucleotide, which may have or include a nucleotide sequence of SEQ ID NO: 10, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 10, or may consist or essentially consist of a nucleotide sequence of SEQ ID NO: 10, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 10, but is not limited thereto.

The variant polypeptide having the activity of the isocitrate lyase of the present disclosure may be a variant polypeptide having the activity of isocitrate lyase in which the amino acid sequences corresponding to positions 336 to 346 of SEQ ID NO: 9 are deleted, but is not limited thereto.

In one embodiment, the variant polypeptide having the activity of the isocitrate lyase of the present disclosure may have a sequence homology of 60% or more and less than 97%, specifically, a sequence homology of 80% or more and less than 97% with the amino acid sequence of SEQ ID NO: 9, but is not limited thereto.

Specifically, the variant polypeptide having the activity of the isocitrate lyase of the present disclosure may include those in which the amino acid sequences corresponding to positions 336 to 346 from the N-terminus of SEQ ID NO: 9 are deleted, in the amino acid sequence having at least 60%, 70%, 75%, 80%, 85%, 90%, 95%, 96%, 97%, 98%, 99%, 99.5%, 99.7% or 99.9% or more homology or identity with the amino acid sequence represented by SEQ ID NO: 9. Additionally, it is apparent that any variant having an amino acid sequence, in which part of the sequence is deleted, modified, substituted, conservatively substituted, or added, may also fall within the scope of the present disclosure as long as it has such a homology or identity and exhibits an efficacy corresponding to that of the variant of the present disclosure.

In another embodiment, the variant polypeptide having the activity of the isocitrate lyase may consist of an amino acid sequence of SEQ ID NO: 11.

Specifically, the variant polypeptide having the activity of the isocitrate lyase of the present disclosure may have or include the amino acid sequence of SEQ ID NO: 11 or an amino acid sequence having at least 60%, 70%, 80%, 85%, 90%, 95%, 96%, 97%, 98%, or 99% or more homology or identity to SEQ ID NO: 11, may consist of the amino acid sequence above, or may essentially consist of the amino acid sequence above.

For example, it may be a case of sequence additions or deletions that do not alter the function of the variant polypeptide of the present disclosure, naturally occurring mutations, silent mutations thereof, or conservative substitutions at the N-terminus, C-terminus and/or within the amino acid sequences.

The polynucleotide encoding the variant polypeptide having the activity of the isocitrate lyase of the present disclosure may include those in which the nucleotide sequences corresponding to positions 1006 to 1038 of SEQ ID NO: 10 are deleted, in the nucleotide sequence having 60%, 70%, 75%, 80%, 85%, 90%, 95%, 96%, 97%, 98%, 99%, 99.5%, 99.7%, or 99.9% or more homology or identity with the sequence of SEQ ID NO: 10, but is not limited thereto. Additionally, it is apparent that any variant having a polynucleotide sequence, in which part of the sequence is deleted, modified, substituted, conservatively substituted, or added, may also fall within the scope of the present disclosure as long as the polynucleotide sequence has such homology or identity and encodes the amino acid sequence of the variant polypeptide having the activity of the isocitrate lyase of the present disclosure.

In another example, the polynucleotide encoding the variant polypeptide having the activity of isocitrate lyase of the present disclosure may have or include a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 12, or may consist or essentially consist of a nucleic acid sequence having 60% or more, 70% or more, 75% or more, 80% or more, 85% or more, 90% or more, 95% or more, 96% or more, 97% or more, 98% or more, and less than 100% homology or identity to SEQ ID NO: 12, but is not limited thereto.

As the microorganism according to any one of the above-described embodiments, the microorganism having an L-glutamic acid-producing ability of the present disclosure may be a microorganism having an increased L-glutamic acid-producing ability by including the variant polypeptide in which the amino acid corresponding to position 351 of SEQ ID NO: 1 is substituted with another amino acid; the variant polypeptide having the activity of the TetR-family transcriptional regulator in which the amino acid corresponding to position 105 of SEQ ID NO: 5 is substituted with another amino acid; and the variant polypeptide having the activity of the isocitrate lyase in which the amino acid sequences corresponding to positions 336 to 346 of SEQ ID NO: 9 are deleted, but is not limited thereto.

As the microorganism according to any one of the above-described embodiments, the microorganism having an L-glutamic acid-producing ability of the present disclosure may be a microorganism having an increased L-glutamic acid-producing ability by including the variant polypeptide in which the amino acid corresponding to position 351 of SEQ ID NO: 1 is substituted with asparagine; the variant polypeptide having the activity of the TetR-family transcriptional regulator in which the amino acid corresponding to position 105 of SEQ ID NO: 5 is substituted with leucine; and the variant polypeptide having the activity of the isocitrate lyase in which the amino acid sequences corresponding to positions 336 to 346 of SEQ ID NO: 9 are deleted, but is not limited thereto.

Meanwhile, the microorganism having an L-glutamic acid-producing ability of the present disclosure may include all of the following: a natural wild-type microorganism itself; a microorganism in which the activity of a polypeptide involved in the L-glutamic acid production mechanism is enhanced or weakened so as to have an improved L-glutamic acid-producing ability; or a microorganism in which the activity of a foreign polypeptide is introduced or enhanced so as to have an improved L-glutamic acid-producing ability.

Meanwhile, although it has been already known that microorganisms of the genus *Corynebacterium* can produce L-glutamic acid, they have a significantly low L-glutamic acid-producing ability, and the genes acting on the production mechanism or the principle of the mechanism have not been fully discovered. Accordingly, the microorganism of the genus *Corynebacterium* having an L-glutamic acid-producing ability of the present disclosure may include all of the following: a natural wild-type microorganism itself; a microorganism of the genus *Corynebacterium* in which the activity of a polypeptide involved in the L-glutamic acid production mechanism is enhanced or weakened so as to have an improved L-glutamic acid-producing ability; or a microorganism of the genus *Corynebacterium* in which the activity of a foreign polypeptide is introduced or enhanced so as to have an improved L-glutamic acid-producing ability.

As used herein, the term "increase" of polypeptide activity means that the activity of a polypeptide is increased, as compared with its endogenous activity thereof. The increase may be used interchangeably with terms such as activation, upregulation, overexpression, enhancement, *etc.*

The increase may include both cases in which an activity not originally possessed is exhibited, or an activity is enhanced compared to the endogenous activity or the activity before modification.

For example, the phrase "activity not originally possessed is exhibited" may refer to the "introduction of a protein", but is not limited thereto. The introduction of a protein means that a gene not originally possessed by a microorganism is expressed in the microorganism, and thus the microorganism exhibits the activity of a particular protein or exhibits an increased or enhanced activity of the corresponding protein compared with the endogenous activity or the activity before modification. For example, the phrase may indicate that a polynucleotide encoding a particular protein is introduced into the chromosome of a microorganism, or a vector containing a polynucleotide encoding a particular protein is introduced into a microorganism, thereby exhibiting the activity of the particular protein.

The "endogenous activity" refers to the activity of a particular polypeptide originally possessed by a parent strain before transformation or a non-modified microorganism when a trait is altered by genetic variation due to a natural or artificial factor. The endogenous activity may be interchangeably used with "activity before modification".

The increase in the activity of the polypeptide, as compared with the endogenous activity, means that the activity of the polypeptide is enhanced, as compared with the activity and/or concentration (expression level) of a particular polypeptide originally possessed by a parent strain before transformation or a non-modified microorganism.

In one example, the increase may mean that the activity of a corresponding protein not originally possessed is exhibited, or the activity or concentration thereof is increased generally by about 1 %, about 10 %, about 25 %, about 50 %, about 75 %, about 100 %, about 150 %, about 200 %, about 300 %, about 400 %, about 500 %, maximum about 1000 %, or about 2000 % or more, based on the activity or concentration of a wild-type protein or an initial microbial strain, but is not limited thereto.

The increase of the activity of the polypeptide may be achieved by introducing a foreign polypeptide or increasing the activity of an endogenous polypeptide. Whether or not the activity of the polypeptide is increased may be confirmed from the activity level of the corresponding polypeptide, expression level thereof, or the increase in the amount of products produced from the corresponding polypeptide.

The increase of the activity of the polypeptide may be applied by various methods well known in the art, and is not limited as long as it can increase the activity of the target polypeptide compared to that of the microorganism before modification. Specifically, genetic engineering and/or protein engineering well known to those skilled in the art, which is a common method of molecular biology, may be used, but the method is not limited thereto (*e.g*., Sitnicka et al. Functional Analysis of Genes. Advances in Cell Biology. 2010, Vol. 2. 1-16, Sambrook et al. Molecular Cloning 2012, *etc*.).

Specifically, the increase of the polypeptide activity of the present disclosure may be achieved by:
1) increasing the intracellular copy number of a polynucleotide encoding the polypeptide;
2) modifying the expression regulatory region of a gene encoding the polypeptide on the chromosome (*e.g*., inducing a modification within the expression regulatory region, replacing with a sequence having a stronger activity, or inserting a sequence having a stronger activity);
3) modifying the nucleotide sequence encoding the initiation codon or 5'-UTR of a gene transcript encoding the polypeptide;
4) modifying the amino acid sequence of the polypeptide such that the activity of the polypeptide is increased;
5) modifying the polynucleotide sequence encoding the polypeptide such that the activity of the polypeptide is increased (*e.g*., modifying the polynucleotide sequence of the polypeptide gene to encode a polypeptide that has been modified to increase the activity of the polypeptide);
6) introducing a foreign polypeptide exhibiting the polypeptide activity or a foreign polynucleotide encoding the same;
7) codon-optimization of a polynucleotide encoding the polypeptide;
8) analyzing the tertiary structure of the polypeptide and thereby selecting and modifying the exposed site, or chemically modifying the same;
9) regulating the cellular localization of the protein (polypeptide); or
10) a combination of two or more selected from above 1) to 9), but is not particularly limited thereto.

For example,
The 1) method of increasing the intracellular copy number of a polynucleotide encoding the polypeptide may be achieved by introducing a vector, which is operably linked to a polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further increase the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of a gene encoding the polypeptide may be achieved, for example, by substituting the initiation codon with another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to increase the activity of the polypeptide; or by replacing the sequence with an amino acid sequence or a polynucleotide sequence modified to have a stronger activity, or an amino acid sequence or a polynucleotide sequence modified to increase the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables the production of the polypeptide, thereby increasing its activity.

The 7) method of codon-optimization of a polynucleotide encoding the polypeptide may be achieved by codon-optimization of an endogenous polynucleotide to increase the transcription or translation within a host cell, or by optimizing the codons thereof such that the optimized transcription and translation of a foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting proteins (polypeptides), but is not limited thereto.

Such enhancement of the polypeptide activity may mean that the activity or concentration of the corresponding polypeptide is increased relative to the activity or concentration of a polypeptide expressed in a wild-type strain or a microbial strain before modification, or that the amount of products produced from the corresponding polypeptide is increased, but is not limited thereto.

As used herein, the term "weakening" of a polypeptide activity is a comprehensive concept including both weakened or no activity compared to its endogenous activity. The weakening may be used interchangeably with terms such as deficiency, inactivation, deletion, disruption, down-regulation, decrease, attenuation, repression, reduction, *etc.*

For example, the weakening may refer to a state in which a protein exhibits activity but is not completely inactivated by deletion, and may mean that the activity of the protein is weakened compared to that of a non-modified microorganism, a wild-type strain, or a parent strain, but is not limited thereto.

For example, the weakening may be, but is not limited to, inactivation. The inactivation may mean that the protein is not expressed at all, or the activity thereof is not exhibited or weakened even when the protein is expressed, as compared to a parent strain or a non-modified strain.

The weakening may also include a case where the polypeptide activity itself is weakened or removed compared to the activity of a polypeptide originally possessed by a microorganism due to mutation of the polynucleotide encoding the polypeptide; a case where the overall level of intracellular polypeptide activity is decreased compared to a natural strain due to the inhibition of expression of a gene encoding the same, or the inhibition of translation into the polypeptide, *etc.;* a case where the gene is not expressed at all; and a case where the activity of the polypeptide is not exhibited even when the gene is expressed.

The weakening of the activity of the polypeptide compared to its endogenous activity means that the activity of the polypeptide is decreased compared to the activity of a particular polypeptide originally possessed by a parent strain before modification or a non-modified microorganism. Whether or not the activity of the polypeptide is weakened may be confirmed from the activity level of the corresponding polypeptide, expression level thereof, or the decrease in the amount of products produced from the corresponding polypeptide.

In one example, the weakening may mean that the activity of the protein is about 100% or less, about 90% or less, about 80% or less, about 70% or less, about 60% or less, about 50% or less, about 40% or less, about 30% or less, about 20% or less, about 10% or less, about 5% or less, or 0%, relative to the activity of the protein of a parent strain before modification or a non-modified microorganism, but is not limited thereto.

For example, the inactivation may mean that the protein is not expressed at all, or the activity thereof is not exhibited or weakened even when the protein is expressed, compared to a non-modified microorganism,
The weakening of the polypeptide activity can be performed by any method known in the art, but the method is not limited thereto, and can be achieved by applying various methods well known in the art (*e.g*., Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide activity of the present disclosure may be achieved by:
1) deleting all or part of a gene encoding the polypeptide;
2) modifying the expression regulatory region (expression regulatory sequence) such that the expression of a gene encoding the polypeptide is weakened;
3) modifying the amino acid sequence constituting the polypeptide such that the polypeptide activity is removed or weakened (*e.g*., deletion/substitution/addition of one or more amino acids on the amino acid sequence);
4) modifying the polynucleotide sequence encoding the polypeptide such that the polypeptide activity is removed or weakened (*e.g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of a gene encoding the polypeptide;
6) introducing an antisense oligonucleotide (*e.g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of apolynucleotide sequence encoding the polypeptide;
9) regulating the cellular localization of the protein (polypeptide); or
10) a combination of two or more selected from the methods 1) to 9) above, but is not particularly limited thereto.

For example,
The 1) method of deleting a part or all of the gene encoding the polypeptide may be achieved by deleting all of the polynucleotide encoding the endogenous target polypeptide within the chromosome, or by replacing the polynucleotide with a polynucleotide having partially deleted nucleotides or with a marker gene.

The method of deleting a part or all of the polynucleotide may be achieved by a method for deleting the polynucleotide by homologous recombination through a vector for chromosomal insertion in the microorganism, or a method, in which light, such as UV rays, or a chemical substance may be used to induce mutations, thereby selecting a target gene-deleted strain from the resulting mutants, but is not limited thereto. The method of deleting a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

The 2) method of modifying the expression regulatory region may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide; or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a modification into the polynucleotide sequence to form a stop codon, but is not limited thereto
The 5) method of modifying the nucleotide sequence encoding the initiation codon or 5'-UTR of a gene encoding the polypeptide may be achieved, for example, by substituting the initiation codon with another initiation codon having a lower polypeptide expression rate than the endogenous initiation codon, but is not limited thereto.

The 6) method of introducing an antisense oligonucleotide (*e.g*., antisense RNA), which binds complementary to the gene transcript encoding the polypeptide can be found in the literature [Weintraub, H. et al., Antisense-RNA as a molecular tool for genetic analysis, Reviews - Trends in Genetics, Vol. 1(1) 1986].

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

The 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting proteins (polypeptides), but is not limited thereto.

Such weakening of the polypeptide activity may mean that the activity or concentration of the corresponding polypeptide is weakened relative to the activity or concentration of a polypeptide expressed in a wild-type strain or a microbial strain before modification, or that the amount of products produced from the corresponding polypeptide is increased, but is not limited thereto.

Even another aspect of the present disclosure provides a method for producing L-glutamic acid, including culturing the microorganism of the present disclosure in a medium.

Specifically, the method for producing L-glutamic acid of the present disclosure may include culturing a microorganism, which includes the variant of the present disclosure, the polynucleotide of the present disclosure, or the vector of the present disclosure, in a medium, but is not limited thereto.

As used herein, the term "cultivation" means that the microorganism of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.* For example, the culture medium for the strains of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.;* and glycerol, propanediol, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for about 10 hours to 160 hours, but is not limited thereto.

The L-glutamic acid produced by the cultivation of the present disclosure may be released into the medium or remain in the cells.

In one embodiment, the method for producing L-glutamic acid of the present disclosure may further include a step of preparing the microorganism of the present disclosure, a step of preparing a medium for culturing the strain, or a combination thereof (regardless of the order, in any order), for example, prior to the culturing step.

In one embodiment, the method for producing L-glutamic acid of the present disclosure may further include a step of recovering L-glutamic acid from the cultured medium (medium on which the cultivation was performed), or from the microorganism of the present disclosure. The recovering step may be further included after the culturing step.

In the recovering step, L-glutamic acid may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and L-glutamic acid can be recovered from the medium or the microorganisms using suitable methods known in the art.

Further, the method for producing L-glutamic acid of the present disclosure may further include a purification step, which may be performed using an appropriate method known in the art. In one example, when the method for producing L-glutamic acid of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the variant polypeptide, polynucleotide and L-glutamic acid, *etc.,* are as described in other aspects above.

Further another aspect of the present disclosure provides a composition for producing L-glutamic acid, including: the variant polypeptide of the present disclosure; a polynucleotide encoding the variant polypeptide; a vector containing the polynucleotide; a microorganism, which includes the variant polypeptide of the present disclosure, a polynucleotide encoding the variant polypeptide, or a vector containing the polynucleotide; a culture product of the microorganism; or a combination of two or more thereof.

The composition of the present disclosure may further include any suitable excipient commonly used in compositions for producing L-glutamic acid, and such excipients may include, for example, preservatives, wetting agents, dispersing agents, suspending agents, buffers, stabilizers, or isotonic agents, *etc.,* but are not limited thereto.

In one embodiment, each component present in the composition of the present disclosure may be contained in a microbiologically effective amount, or in an amount that can be appropriately present in the composition for production.

In the composition of the present disclosure, the variant polypeptide, polynucleotide and L-glutamic acid, *etc.,* are as described in other aspects above.

Still further another aspect of the present disclosure provides the use of the variant polypeptide of the present disclosure for the production of L-glutamic acid.

The variant polypeptide of the present disclosure, L-glutamic acid, *etc.,* are as described in other aspects above.

Still further another aspect of the present disclosure provides the use of the microorganism of the present disclosure for the production of L-glutamic acid.

The variant polypeptide of the present disclosure, L-glutamic acid, *etc.,* are as described in other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1. Selection of Mutant Strain for Increasing L-Glutamic Acid Producing Ability through Artificial Mutation

### Example 1-1. Induction of Artificial Mutation by UV Irradiation

In order to select mutant strains with improved L-glutamic acid-producing ability, which is a target product of fermentation, the wild-type *Corynebacterium glutamicum* (ATCC13869) was first plated on a nutrient medium containing agar and cultured at 30°C for 16 hours. Hundreds of the thus-obtained colonies were irradiated with UV at room temperature to induce a random mutation on the genome in the strain.

### Example 1-2. Experiment on Fermentation Titer of Mutation-Inducting Strain and Selection of the Strain

The experiment on fermentation titer of the mutant strains, in which the random mutation had been induced in Example 1-1, was carried out.

Each colony was sub-cultured in the nutrient medium, and then cultured in a fermentation medium for 5 hours. Thereafter, 25% tween 40 was added to each medium at a concentration of 0.4%, and then each colony was cultured again for 32 hours.

### <Nutrient Medium>

Glucose 1%, meat juice 0.5%, polypeptone 1%, sodium chloride 0.25%, yeast extract 0.5%, agar 2%, urea 0.2%, pH 7.2

### <Fermentation Medium>

Glucose 6%, calcium carbonate 5%, ammonium sulfate 2.25%, potassium monophosphate 0.1%, magnesium sulfate 0.04%, iron sulfate 10 mg/L, biotin 0.3 mg/L, thiamine hydrochloride 0.2 mg/L

Each of the colonies was cultured under the conditions above, and then mutant strains that produced L-glutamic acid in an amount equal to or greater than that produced by the wild-type *Corynebacterium glutamicum* (ATCC13869) were selected. Thereafter, with respect to the selected mutant strains, the concentration of L-glutamic acid was measured by HPLC. The measured concentrations of L-glutamic acid are shown in Table 1 below.

**[Table 1]**

| Strain | L-Glutamic acid (g/L) |
|---|---|
| ATCC13869 | 7.2 |
| ATCC13869-C1 | 8.2 |
| ATCC13869-C2 | 7.1 |
| ATCC13869-C3 | 8.4 |
| ATCC13869-C4 | 8.9 |
| ATCC13869-C5 | 8.6 |
| ATCC13869-C6 | 7.9 |
| ATCC13869-C7 | 9.0 |
| ATCC13869-C8 | 8.5 |
| ATCC13869-C9 | 7.6 |
| ATCC13869-C10 | 6.4 |
| ATCC13869-C11 | 7.0 |
| ATCC13869-C12 | 8.2 |
| ATCC13869-C13 | 7.4 |
| ATCC13869-C14 | 7.9 |
| ATCC13869-C15 | 8.1 |

Based on Table 1 above, "ATCC13869-C4" and "ATCC13869-C7" were selected as the mutant strains with increased L-glutamic acid production amounts compared to the wild-type strain.

### Example 2. Confirmation of Mutation Through Gene Sequencing

In order to confirm the gene mutation of the mutant strains, genes of the ATCC13869-C4 and ATCC13869-C7 strains selected in Example 1-2 were compared with those of the wild-type strain.

As a result, it was found that the ATCC13869-C4 and ATCC13869-C9 strains contained the same mutation (wherein the nucleotide at position 1052 of the polynucleotide sequence represented by SEQ ID NO: 2 is substituted with A) at a specific position of the *cgmA* gene (SEQ ID NO: 2) encoding the major facilitator superfamily transporter.

Accordingly, in Examples 3 and 4, attempts were made to confirm whether the mutation above had an effect on the production amount of L-glutamic acid in the microorganism of the genus *Corynebacterium.*

### Example 3. Preparation of Strains Introduced with Mutation and Confirmation of Production Amount of L-Glutamic Acid - 1

### Example 3-1. Preparation of Strains Introduced with Mutation - 1

An attempt was made to prepare a mutant strain introduced with the mutation confirmed in Example 2. Specifically, a vector for gene substitution was prepared in order to substitute the 351^{st} isoleucine of the major facilitator superfamily transporter represented by SEQ ID NO: 1, which is contained in the strain, with asparagine, by introducing the mutation (wherein the 1052^{nd} nucleotide of the polynucleotide sequence represented by SEQ ID NO: 2 is substituted with A) into the wild-type *Corynebacterium glutamicum* (ATCC13869 and ATCC13032). The gene fragments for preparing the vector were obtained by PCR using ATCC13869 genomic DNA as a template. Based on information on genes and adjacent sequences of the *Corynebacterium glutamicum* (ATCC13869) registered in the National Institutes of Health GenBank (NIH GenBank), primers including the polynucleotides of SEQ ID NOS: 13, 14, 15, and 16 were prepared.

PCR was carried out by denaturation at 95°C for 5 minutes, followed by 30 cycles of denaturation at 95°C for 20 seconds, annealing at 55°C for 20 seconds, and polymerization at 72°C for 30 seconds, and then polymerization at 72°C for 5 minutes. More specifically, the polynucleotide (500 bp) amplified using the primers of SEQ ID NOS: 13 and 14, and the polynucleotide (500 bp) amplified using the primers of SEQ ID NOS: 15 and 16 were obtained. The obtained two DNA fragments were ligated to the vector pDZ (Korean Patent No. 10-0924065 and International Publication No. 2008-033001), which had been digested with restriction enzymes BamHI and Sall, using an infusion enzyme, and thereby a vector for gene substitution was prepared. The prepared vector was named as "pDZ-cgmA(I351N)". The information on the primer sequences used for the vector preparation is shown in Table 2 below.

**[Table 2]**

| SEQ ID NO: | Primer | Sequence (5' to 3') |
|---|---|---|
| 13 | CgmA(I351N)-AF | CGGTACCCGGGGATCCGTTCTGTGGCAATTCTTGG |
| 14 | CgmA(I351N)-AR | AATACACAGGGCGTTGCCGACTGCCGTGGCAA |
| 15 | CgmA(I351N)-BF | ACGGCAGTCGGCAACGCCCTGTGTATTTGGGGC |
| 16 | CgmA(I351N)-;BR | ATGCCTGCAGGTCGACAAGCAACTCGTGCATGCCT A |

Subsequently, the vector for gene substitution was transformed into the wild-type strain by homologous recombination on the chromosome (van der Rest et al., Appl Microbiol Biotechnol 52:541-545, 1999).

The strains in which the vector was inserted on the chromosome by recombination of homologous sequences were selected from a medium containing 25 mg/L kanamycin. Thereafter, the transformed strains of *Corynebacterium glutamicum* for which secondary recombination had been completed were subjected to gene sequencing analysis, and as a result, it was confirmed that the target mutation was introduced into the strains, and the resulting strains were named "ATCC13869::cgmA(I351N)" and "ATCC13032::cgmA(I351N)".

### Example 3-2. Confirmation of L-Glutamic Acid Production Amount - 1

The mutant strains of ATCC13869::cgmA(I351N) and ATCC13032::cgmA(I351N) prepared in Example 3-1, and their wild-type *Corynebacterium glutamicum* (ATCC13869 and ATCC13032) strains were cultured in the same manner as in Example 1-2.

After completion of the culture, the L-glutamic acid concentration in each medium was measured. The measured L-glutamic acid concentrations are shown in Table 3 below.

**[Table 3]**

| Strain | L-Glutamic acid (g/L) |
|---|---|
| ATCC13869 | 7.0 |
| ATCC13869:: cgmA(I351N) | 8.5 |
| ATCC13032 | 3.6 |
| ATCC13032:: cgmA(I351N) | 4.4 |

As shown in Table 3, it was confirmed that the concentration of L-glutamic acid produced by the *Corynebacterium glutamicum* ATCC13869::cgmA(I351N) strain, into which the mutation was introduced, was about 1.5 g/L (about 21%) higher compared to the concentration of L-glutamic acid produced by the wild-type *Corynebacterium glutamicum* ATCC13869.

Additionally, it was confirmed that the concentration of L-glutamic acid produced by the *Corynebacterium glutamicum* ATCC13032::cgmA(I351N) strain, into which the mutation was introduced, was about 0.8 g/L (about 22%) higher compared to the concentration of L-glutamic acid produced by the wild-type *Corynebacterium glutamicum* ATCC13032.

That is, it was confirmed that the variant of the present disclosure increased the L-glutamic acid-producing ability of the microorganism.

### Example 4. Preparation of Strains Introduced with Mutation and Confirmation of Production Amount of L-Glutamic Acid - 2

### Example 4-1. Preparation of Strains Introduced with Mutation - 2

An attempt was made to further introduce a variant of TetR-family transcriptional regulator (mmpLR) into the mutant strains of ATCC13869::cgmA(1351N) and ATCC13032::cgmA(I351N) prepared in Example 3-1.

Specifically, a vector for gene substitution was prepared in order to substitute the 105^{th} proline of the TetR-family transcriptional regulator represented by SEQ ID NO: 5, which is contained in the strain, with leucine, by introducing the mmpLR mutation (wherein the 314^{th} nucleotide of the polynucleotide sequence represented by SEQ ID NO: 6 is substituted with T) into the wild-type *Corynebacterium glutamicum* (ATCC13869 and ATCC13032). The gene fragments for preparing the vector were obtained by PCR using ATCC13869 genomic DNA as a template. Based on information on genes and adjacent sequences of the *Corynebacterium glutamicum* (ATCC13869) registered in the National Institutes of Health GenBank (NIH GenBank), primers including the polynucleotides of SEQ ID NOS: 17, 18, 19, and 20 were prepared.

PCR was carried out by denaturation at 95°C for 5 minutes, followed by 30 cycles of denaturation at 95°C for 20 seconds, annealing at 55°C for 20 seconds, and polymerization at 72°C for 30 seconds, and then polymerization at 72°C for 5 minutes. More specifically, the polynucleotide (500 bp) amplified using the primers of SEQ ID NOS: 17 and 18, and the polynucleotide (500 bp) amplified using the primers of SEQ ID NOS: 19 and 20 were obtained. The thus-obtained two DNA fragments were ligated to the vector pDZ (Korean Patent No. 10-0924065 and International Publication No. 2008-033001), which had been digested with restriction enzymes BamHI and Sall, using an infusion enzyme, and thereby a vector for gene substitution was prepared. The prepared vector was named as "pDZ-mmpLR(P105L)". The information on the primer sequences used for the vector preparation is shown in Table 4 below.

**[Table 4]**

| SEQ ID NO: | Primer | Sequence (5' to 3') |
|---|---|---|
| 17 | mmpLR(P105L)-AF | CGGTACCCGGGGATCCCTTGATGACAAGGCT TGG |
| 18 | mmpLR(P105L)-AR | GATAACACACTTGATGAGGTCAAGATTTTCAA GG |
| 19 | mmpLR(P105L)-BF | TCATCAAGTGTGTTATCGATAAGGAGCGACTC A |
| 20 | mmpLR(P105L)-BR | ATGCCTGCAGGTCGACTGCAGGGCTTCGGGC TTG |

Subseuqenlty, the vector for gene substitution was transformed into the ATCC13869::cgmA(I351N) and ATCC13032::cgmA(I351N) strains by homologous recombination on the chromosome. The strains in which the vector was inserted on the chromosome by recombination of homologous sequences were selected from a medium containing 25 mg/L kanamycin. Thereafter, the transformed strains of *Corynebacterium glutamicum* for which secondary recombination had been completed were subjected to gene sequencing analysis, and as a result, it was confirmed that the target mutation was introduced into the strains, and the resulting strains were named "ATCC13869::cgmA(I351N)_mmpLR(P105L))" and "ATCC13032::cgmA(I351N)_mmpLR(P105L)".

### Example 4-2. Confirmation of L-Glutamic Acid Production Amount - 2

The mutant strains of ATCC13869::cgmA(I351N)_mmpLR(P105L) and ATCC13032::cgmA(I351N)_mmpLR(P105L) prepared in Example 4-1, and their wild-type *Corynebacterium glutamicum* (ATCC13869 and ATCC13032) strains were cultured in the same manner as in Example 1-2.

After completion of the culture, the L-glutamic acid concentration in each medium was measured. The measured L-glutamic acid concentrations are shown in Table 5 below.

**[Table 5]**

| Strain | L-Glutamic acid (g/L) |
|---|---|
| ATCC13869 | 7.0 |
| ATCC13869:: cgmA(1351N)_mmpLR(P105L) | 9.4 |
| ATCC13032 | 3.6 |
| ATCC13032:: cgmA(1351N)_mmpLR(P105L) | 4.7 |

As shown in Table 5, it was confirmed that the concentration of L-glutamic acid produced by the *Corynebacterium glutamicum* ATCC13869::cgmA(I351N)_mmpLR(P105L) strain, into which the mutation was introduced, was about 2.4 g/L (about 34%) higher compared to the concentration of L-glutamic acid produced by the wild-type *Corynebacterium glutamicum* ATCC13869.

Additionally, it was confirmed that the concentration of L-glutamic acid produced by the *Corynebacterium glutamicum* ATCC13032::cgmA(I351N)_mmpLR(P105L) strain, into which the mutation was introduced, was about 1.1 g/L (about 31%) higher compared to the concentration of L-glutamic acid produced by the wild-type *Corynebacterium glutamicum* ATCC13032.

That is, it was confirmed that when the variant polypeptide having the activity of the TetR-family transcriptional regulator was additionally introduced into the microorganisms including the variant polypeptide of the present disclosure, the L-glutamic acid-producing ability was further increased.

### Example 5. Preparation of Strains Introduced with Mutation and Confirmation of Production Amount of L-Glutamic Acid - 3

### Example 5-1. Preparation of Strains Introduced with Mutation - 3

An attempt was made to further introduce a variant of isocitrate lyase (aceA) into the mutant strains of ATCC13869::cgmA(I351N)_mmpLR(P105L) and ATCC13032::cgmA(I351N)_mmpLR(P105L) prepared in Example 4-1.

Specifically, a vector for gene substitution was prepared in order to delete from lysine corresponding to position 336 to glutamine corresponding to position 346 of the isocitrate lyase represented by SEQ ID NO: 9, which is contained in the strain, by introducing the aceA mutation (wherein the nucleotide sequences corresponding to positions 1006 to 1038 of the polynucleotide sequence represented by SEQ ID NO: 10 are deleted) into the wild-type *Corynebacterium glutamicum* (ATCC13869 and ATCC13032). The gene fragments for preparing the vector were obtained by PCR using ATCC13869 genomic DNA as a template. Based on information on genes and adjacent sequences of the *Corynebacterium glutamicum* (ATCC13869) registered in the National Institutes of Health GenBank (NIH GenBank), primers including the polynucleotides of SEQ ID NOS: 21, 22, 23, and 24 were prepared.

PCR was carried out by denaturation at 95°C for 5 minutes, followed by 30 cycles of denaturation at 95°C for 20 seconds, annealing at 55°C for 20 seconds, and polymerization at 72°C for 30 seconds, and then polymerization at 72°C for 5 minutes. More specifically, the polynucleotide (500 bp) amplified using the primers of SEQ ID NOS: 21 and 22, and the polynucleotide (500 bp) amplified using the primers of SEQ ID NOS: 23 and 24 were obtained. The thus-obtained two DNA fragments were ligated to the vector pDZ (Korean Patent No. 10-0924065 and International Publication No. 2008-033001), which had been digested with restriction enzymes BamHI and Sall, using an infusion enzyme, and thereby a vector for gene substitution was prepared. The prepared vector was named as "pDZ-aceA_deletion". The information on the primer sequences used for the vector preparation is shown in Table 6 below.

**[Table 6]**

| SEQ ID NO: | Primer | Sequence (5' to 3)' |
|---|---|---|
| 21 | aceA_deletion-AF | CGGTACCCGGGGATCC AATGATCGCAGCTGGCGC |
| 22 | aceA_deletion -AR | GGGTGATGAACTGGAACTTAGCGATCTCATCTG CC |
| 23 | aceA_deletion -BF | GTTCCAGTTCATCACCCTCGCAGGCTTCCACTC CC |
| 24 | aceA_deletion -BR | ATGCCTGCAGGTCGAC GACGGTGCGGGAAATAGAC |

Subsequently, the vector for gene substitution was transformed into the ATCC13869::cgmA(I351N)_mmpLR(P105L) and ATCC13032::cgmA(I351N)_mmpLR(P105L) strains by homologous recombination on the chromosome. The strains in which the vector was inserted on the chromosome by recombination of homologous sequences were selected from a medium containing 25 mg/L kanamycin. Thereafter, the transformed strains of *Corynebacterium glutamicum* for which secondary recombination had been completed were subjected to gene sequencing analysis, and as a result, it was confirmed that the target mutation was introduced into the strains, and the resulting strains were named "ATCC13869::cgmA(I351N)_mmpLR(P105L)_aceA_deletion" and "ATCC13032::cgmA(1351 N)_mmpLR(P105L) aceA deletion".

### Example 5-2. Confirmation of L-Glutamic Acid Production Amount - 3

The mutant strains of ATCC13869::cgmA(I351N)_mmpLR(P105L)_aceA_deletion and ATCC13032::cgmA(I351N)_mmpLR(P105L)_aceA_deletion prepared in Example 5-1, and their wild-type *Corynebacterium glutamicum* (ATCC13869 and ATCC13032) strains were cultured in the same manner as in Example 1-2.

After completion of the culture, the L-glutamic acid concentration in each medium was measured. The measured L-glutamic acid concentrations are shown in Table 7 below.

**[Table 7]**

| Strain | L-Glutamic acid (g/L) |
|---|---|
| ATCC13869 | 7.0 |
| ATCC13869:: cgmA(1351N)_mmpLR(P105L)_aceA_deletion | 10.3 |
| ATCC13032 | 3.6 |
| ATCC13032:: cgmA(1351N)_mmpLR(P105L)_aceA_deletion | 5.2 |

As shown in Table 7, it was confirmed that the concentration of L-glutamic acid produced by the *Corynebacterium glutamicum* ATCC13869::cgmA(I351N)_mmpLR(P105L)_aceA_deletion strain, into which the mutation was introduced, was about 3.3 g/L (about 47%) higher compared to the concentration of L-glutamic acid produced by the wild-type *Corynebacterium glutamicum* ATCC13869.

Additionally, it was confirmed that the concentration of L-glutamic acid produced by the *Corynebacterium glutamicum* ATCC13032::cgmA(I351N)_mmpLR(P105L)_aceA_deletion strain, into which the mutation was introduced, was about 1.6 g/L (about 44%) higher compared to the concentration of L-glutamic acid produced by the wild-type *Corynebacterium glutamicum* ATCC13032.

That is, it was confirmed that when the variant polypeptide having the activity of the TetR-family transcriptional regulator and the variant polypeptide having the activity of the isocitrate lyase were additionally introduced into the microorganisms including the variant polypeptide of the present disclosure, the L-glutamic acid-producing ability was further increased.

### Example 6. Confirmation of Production Amount of L-Glutamic Acid in KFCC11074 Strain Introduced with Mutation

### Example 6-1. Preparation of Strain Introduced with Mutation - 1

In order to confirm whether the mutation had the same effect in a strain with an increased L-glutamic acid-producing ability, in addition to the wild-type strain, an attempt was made to introduce the mutation into KFCC11074 strain (Korean Patent No. 10-0292299), which is well known as a L-glutamic acid-producing strain.

Specifically, the pDZ-cgmA(I351N) vector prepared in Example 3-1 was transformed into KFCC11074 strain by homologous recombination on the chromosome (van der Rest et al., Appl Microbiol Biotechnol 52:541-545, 1999). The strain in which the vector was inserted on the chromosome by recombination of homologous sequences was selected from a medium containing 25 mg/L kanamycin. Thereafter, the transformed strain of *Corynebacterium glutamicum* for which secondary recombination had been completed was subjected to gene sequencing analysis, and as a result, it was confirmed that the target mutation was introduced into the strain, and the resulting strain was named "KFCC11074_cgmA(I351N)".

### Example 6-2. Preparation of Strain Introduced with Mutation - 2

An attempt was made to further introduce a variant of TetR-family transcriptional regulator (mmpLR) into the mutant strain of KFCC11074_cgmA(I351N) prepared in Example 6-1.

Specifically, the pDZ-mmpLR(P105L) vector prepared in Example 4-1 was transformed into KFCC11074_cgmA(I351N) strain by homologous recombination on the chromosome (van der Rest et al., Appl Microbiol Biotechnol 52:541-545, 1999). The strain in which the vector was inserted on the chromosome by recombination of homologous sequences was selected from a medium containing 25 mg/L kanamycin. Thereafter, the transformed strain of *Corynebacterium glutamicum* for which secondary recombination had been completed was subjected to gene sequencing analysis, and as a result, it was confirmed that the target mutation was introduced into the strain, and the resulting strain was named "KFCC11074_cgmA(I351N)_mmpLR(P105L)".

### Example 6-3. Preparation of Strain Introduced with Mutation - 3

An attempt was made to further introduce a variant of isocitrate lyase (aceA) into the mutant strain of KFCC11074_cgmA(I351N)_mmpLR(P105L) prepared in Example 6-2.

Specifically, the pDZ-aceA_deletion vector prepared in Example 5-1 was transformed into KFCC11074_cgmA(I351N)_mmpLR(P105L) strain by homologous recombination on the chromosome (van der Rest et al., Appl Microbiol Biotechnol 52:541-545, 1999). The strain in which the vector was inserted on the chromosome by recombination of homologous sequences was selected from a medium containing 25 mg/L kanamycin. Thereafter, the transformed strain of *Corynebacterium glutamicum* for which secondary recombination had been completed was subjected to gene sequencing analysis, and as a result, it was confirmed that the target mutation was introduced into the strain, and the resulting strain was named "KFCC11074_cgmA(I351N)_mmpLR(P105L)_aceA_deletion".

### Example 7. Confirmation of Production Amount of L-Glutamic Acid in KFCC11074 Strain Introduced with Mutation

### Example 7-1. Confirmation of L-Glutamic Acid Production Amount - 1

The mutant strain of KFCC11074_cgmA(I351N) prepared in Example 6-1, and its wild-type KFCC11074 strain were cultured in the same manner as in Example 1-2.

After completion of the culture, the L-glutamic acid concentration in each medium was measured. The measured L-glutamic acid concentrations are shown in Table 8 below.

**[Table 8]**

| Strain | L-Glutamic acid (g/L) |
|---|---|
| KFCC11074 | 5.6 |
| KFCC11074_cgmA(I351N) | 6.9 |

As shown in Table 8, it was confirmed that the concentration of L-glutamic acid produced by the *Corynebacterium glutamicum* KFCC11074_cgmA(I351N) strain, into which the mutation was introduced, was about 1.3 g/L (about 23%) higher compared to the concentration of L-glutamic acid produced by the *Corynebacterium glutamicum* KFCC11074, into which the mutation was not introduced.

That is, it was confirmed that the mutation of the present disclosure increased the L-glutamic acid-producing ability of the microorganisms even in the strains with an increased L-glutamic acid-producing ability.

### Example 7-2. Confirmation of L-Glutamic Acid Production Amount - 2

The mutant strains of KFCC11074_cgmA(1351N) and KFCC11074_cgmA(I351N)_mmpLR(P105L) prepared in Examples 6-1 and 6-2, respectively, and their wild-type KFCC11074 strain were cultured in the same manner as in Example 1-2.

After completion of the culture, the L-glutamic acid concentration in each medium was measured. The measured L-glutamic acid concentrations are shown in Table 9 below.

**[Table 9]**

| Strain | L-Glutamic acid (g/L) |
|---|---|
| KFCC11074 | 5.6 |
| KFCC11074_cgmA(I351N)_ mmpLR(P105L) | 7.8 |

As shown in Table 9, it was confirmed that the concentration of L-glutamic acid produced by the *Corynebacterium glutamicum* KFCC11074_cgmA(I351N)_mmpLR(P105L) strain, into which the mutation was introduced, was about 2.2 g/L (about 39%) higher compared to the concentration of L-glutamic acid produced by the *Corynebacterium glutamicum* KFCC11074, into which the mutation was not introduced.

That is, it was confirmed that when the variant polypeptide having the activity of the TetR-family transcriptional regulator was additionally introduced into the microorganisms including the variant polypeptide of the present disclosure, the L-glutamic acid-producing ability of the microorganisms was further increased even in the strains with an increased L-glutamic acid-producing ability.

### Example 7-3. Confirmation of L-Glutamic Acid Production Amount - 3

The mutant strains of KFCC11074_cgmA(1351N), KFCC11074_cgmA(I351N)_mmpLR(P105L) and KFCC11074_cgmA(I351N)_mmpLR(P105L)_aceA_deletion prepared in Examples 6-1, 6-2, and 6-3, respectively, and their wild-type KFCC11074 strain were cultured in the same manner as in Example 1-2.

After completion of the culture, the L-glutamic acid concentration in each medium was measured. The measured L-glutamic acid concentrations are shown in Table 10 below.

**[Table 10]**

| Strain | L-Glutamic acid (g/L) |
|---|---|
| KFCC11074 | 5.6 |
| KFCC11074_cgmA(I351N)_ mmpLR(P105L)_aceA_delet ion | 8.5 |

As shown in Table 10, it was confirmed that the concentration of L-glutamic acid produced by the *Corynebacterium glutamicum* KFCC11074_cgmA(1351N)_mmpLR(P105L)_aceA_deletion strain, into which the mutation was introduced, was about 3.1 g/L (about 52%) higher compared to the concentration of L-glutamic acid produced by the *Corynebacterium glutamicum* KFCC11074, into which the mutation was not introduced.

That is, it was confirmed that when the variant polypeptide having the activity of the TetR-family transcriptional regulator and the variant polypeptide having the activity of the isocitrate lyase were additionally introduced into the microorganisms including the variant polypeptide of the present disclosure, the L-glutamic acid-producing ability of the microorganisms was further increased even in the strains with an increased L-glutamic acid-producing ability.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A variant polypeptide, in which the amino acid corresponding to position 351 of SEQ ID NO: 1 is substituted with another amino acid.

2. The variant polypeptide of claim 1, wherein the variant polypeptide is one in which the amino acid corresponding to position 351 of SEQ ID NO: 1 is substituted with asparagine.

3. The variant polypeptide of claim 1, wherein the variant polypeptide comprises a sequence having 80% or more sequence identity with the amino acid sequence of SEQ **ID** NO: 1.

4. A polynucleotide encoding the variant polypeptide of any one of claims 1 to 3.

5. A microorganism comprising the variant polypeptide of any one of claims 1 to 3, a polynucleotide encoding the variant polypeptide, or a vector comprising the polynucleotide.

6. The microorganism of claim 5, wherein the microorganism further comprises any one or more variant polypeptides selected from the group consisting of (a) and (b); a polynucleotide encoding the variant polypeptide; or a vector comprising the polynucleotide:
wherein (a) a variant polypeptide having the activity of a TetR-family transcriptional regulator in which the amino acid corresponding to position 105 of SEQ ID NO: 5 is substituted with another amino acid; and
(b) a variant polypeptide having the activity of isocitrate lyase in which the amino acid sequences corresponding to positions 336 to 346 of SEQ ID NO: 9 are deleted.

7. The microorganism of claim 6, wherein (a) the variant polypeptide having the activity of a TetR-family transcriptional regulator is a variant polypeptide having the activity of a TetR-family transcriptional regulator in which the amino acid corresponding to position 105 of SEQ ID NO: 5 is substituted with leucine.

8. The microorganism of claim 6, wherein (a) the variant polypeptide having the activity of a TetR-family transcriptional regulator consists of an amino acid sequence of SEQ ID NO: 7; and
(b) the variant polypeptide having the activity of isocitrate lyase consists of an amino acid sequence of SEQ ID NO: 11.

9. The microorganism of claim 5, wherein the microorganism has an increased L-glutamic acid-producing ability compared to a microorganism comprising a polypeptide of SEQ ID NO: 1 or a polynucleotide encoding the same.

10. The microorganism of claim 5, wherein the microorganism is a microorganism of the genus *Corynebacterium.*

11. The microorganism of claim 10, wherein the microorganism of the genus *Corynebacterium* is *Corynebacterium glutamicum.*

12. A method for producing L-glutamic acid, comprising culturing the microorganism of claim 5 in a medium.

13. The method of claim 12, further comprising recovering L-glutamic acid from the cultured microorganism, a culture product of the microorganism, a fermented product of the microorganism, or the cultured medium.

14. A composition for producing L-glutamic acid, comprising: the variant polypeptide of any one or claims 1 to 3; a polynucleotide encoding the variant polypeptide; a vector comprising the polynucleotide; a microorganism, which comprises the variant polypeptide, a polynucleotide encoding the variant polypeptide, or a vector comprising the polynucleotide; a culture product of the microorganism; or a combination of two or more thereof.

15. Use of the variant polypeptide of any one or claims 1 to 3; a polynucleotide encoding the variant polypeptide; a vector comprising the polynucleotide; a microorganism, which comprises the variant polypeptide, a polynucleotide encoding the variant polypeptide, or a vector comprising the polynucleotide; a culture product of the microorganism; or a combination of two or more thereof for the production of L-glutamic acid.
